# EUROPEAN PATENT APPLICATION

(11) **EP 4 725 545 A1**
(43) Date of publication of application: **15.04.2026**
(21) Application number: 24205464.1
(22) Date of filing: 09.10.2024
(51) Int. Cl.: A61N 1/39

(54) **ENHANCED RESCUE PROTOCOL MANAGEMENT OF A CARDIOPULMONARY RESUSCITATION**

(71) Applicant: Koninklijke Philips N.V., 5656 AG Eindhoven (NL)
(72) Inventor: BURGETT, Kevin Scott, Eindhoven (NL); LIU, Chenguang, Eindhoven (NL); GEHMAN, Stacy Earl, 5656AG Eindhoven (NL); JORGENSON, Dawn Blilie, Eindhoven (NL)
(74) Representative: Philips Intellectual Property & Standards

(57) **Abstract**

A defibrillation method involving systematically operating a defibrillator (200) between a scheduled operation mode and a custom operation mode. The scheduled operation mode includes an uninterruptible CPR protocol of a fixed CPRtime period and a scheduled shock protocol. The custom operation mode includes an interruptible CPR protocol of a variable time period and a custom shock protocol. An initial execution of the custom operation mode by the defibrillator (200) is disabled by a defibrillation controller (204) prior to a delivery of an initial defibrillating shock to a heart of a patient by the defibrillator (200), and the initial execution of the custom operation mode by the defibrillator (200) is enabled by the defibrillation controller (204) subsequent to the delivery of the initial defibrillating shock by the defibrillator (200) to the heart of the patient.

## Description

### FIELD OF THE INVENTION

The present disclosure generally relates to a resuscitation rescue effort by a responder or responders of a patient experiencing cardiac arrest. The present disclosure particularly relates to a defibrillator management of the resuscitation rescue effort by a responder or responders of a patient experiencing cardiac arrest to facilitate a successful rescue outcome of the patient.

### BACKGROUND OF THE INVENTION

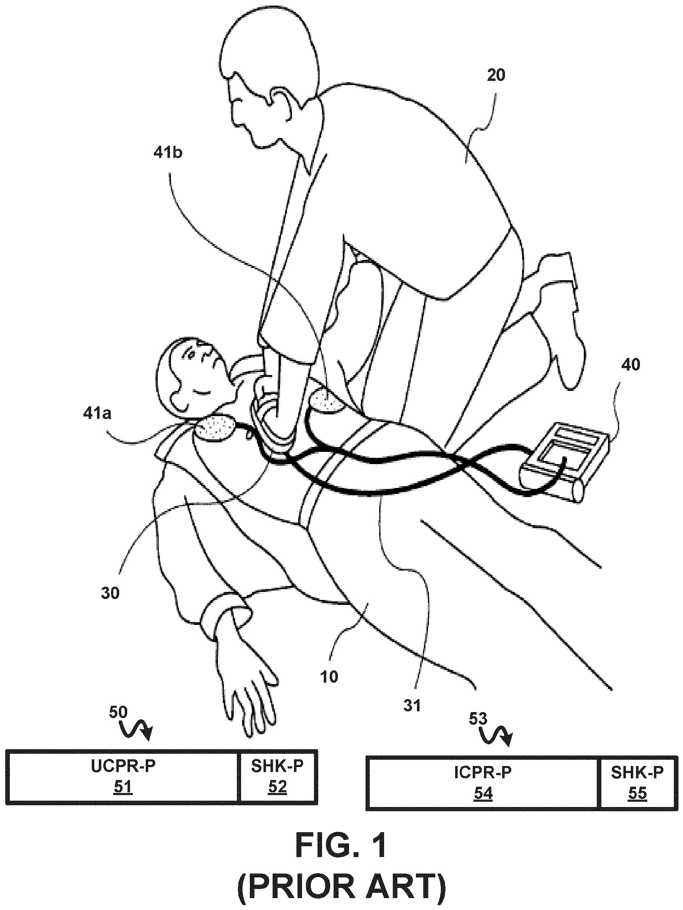

As shown in FIG. 1, a defibrillator 40 is attached to a patient 10 by electrodes 41a and 41b. Defibrillator 40, as known in the art of the present disclosure, can be used to deliver defibrillating shocks to patient 10 when patient 10 is suffering from cardiac arrest. More specifically, defibrillator 40 can deliver a high-voltage impulse to the heart of patient 10 in order to restore organized rhythm and contractile function for the heart of patient 10 when patient 10 is experiencing an arrhythmia (e.g., ventricular fibrillation (VF) or ventricular tachycardia (VT)) that is not accompanied by spontaneous circulation. There are several classes of defibrillators, including manual defibrillators, implantable defibrillators, and automatic/semi-automatic external defibrillators (AEDs). AEDs differ from manual defibrillators in that AEDs can automatically analyze cardiac rhythm(s) of an electrocardiogram (ECG) to determine if defibrillation is necessary. In semi-automatic AED designs, the responder(s) are prompted to press a shock button to deliver the defibrillating shock to the patient when a shock is advised by the AED. The electrodes 41a and 41b are applied across the chest of the patient 10 by a responder 20 as shown in order to acquire an ECG signal from the patient's heart. Defibrillator 40 then analyzes the ECG signal for signs of arrhythmia. If VF or another shockable cardiac rhythm is detected,
then defibrillator 40 signals responder 20 that a shock is advised. After the AED detecting VF or another shockable cardiac rhythm and signaling the responder 20 of such detection, then responder 20 presses a shock button on defibrillator 40 to deliver a defibrillating shock in an attempt to resuscitate patient 10.

As shown, a CPR coaching device 30 can be coupled to defibrillator 40 by an electrical cable 31 to provide defibrillator 40 with physiological information obtained by sensors contained in the CPR coaching device 30 as known in the art of the present disclosure. Electrical cable 31 provides power for electronic components in CPR coaching device 30 and couples compression related signals to defibrillator 40 (e.g., compression depth, compression rate, chest release and recoil), whereby the physiological information indicated by the signals can be used to issue audible CPR instructions through the loudspeaker of defibrillator 40.

As known in the art of the present disclosure, defibrillator 40 can be operated in a scheduled operation mode 50 or a custom operation mode 53. In operation, both scheduled operation mode 50 and custom operation mode 53 of defibrillator 40 include a CPR protocol specifying rule(s)/guideline(s) for responder 20 to administer CPR to the heart of patient 10, and a shock protocol for specifying rule(s)/guideline(s) for a delivery (automatic, semi-automatic or manual) of a defibrillating shock to a heart of patient 10.

More particularly, an uninterruptible CPR protocol 51 of the scheduled operation mode 50 is executed over a fixed CPR time period whereby, upon a timely termination of the fixed CPR time period, a scheduled shock protocol 52 of the scheduled operation mode 50 is executed for a delivery of a defibrillating shock in the circumstance that a shockable cardiac rhythm is detected in a corrupt ECG waveform of the heart of patient 10 during the fixed CPR time period of uninterruptible CPR protocol 51.

Scheduled shock protocol 52 can also be executable for a delivery of a defibrillating shock in the circumstance that a shockable cardiac rhythm is not detected in a corrupt ECG waveform of the heart of patient 10 during the fixed CPR time period of uninterruptible CPR protocol 51and a shockable cardiac rhythm is detected in a clean ECG waveform of the heart of patient 10 succeeding the termination of the fixed CPR time period of uninterruptible CPR protocol 51.

Conversely, an interruptible CPR protocol 54 of the custom operation mode 53 is executed over a terminable CPR time period whereby the terminable CPR time period is pre-timely terminated in the circumstance that a shockable cardiac rhythm is detected in a corrupt ECG waveform of the heart of patient 10 during the terminable CPR time period of interruptible CPR protocol 54, and a custom shock protocol 55 of the custom operation mode 53 is immediately prompted for an execution of a delivery of defibrillating shock to the heart of patient 10.

Custom shock protocol 55 can also be executable for a delivery of a defibrillating shock in the circumstances that a shockable cardiac rhythm is not detected in a corrupt ECG waveform of the heart of patient 10 during the terminable CPR time period of interruptible CPR protocol 54 and a shockable cardiac rhythm is detected in a clean ECG waveform of the heart of patient 10 succeeding the timely termination of the terminable CPR time period of interruptible CPR protocol 54.

The defibrillator industry is constantly striving to optimize a systematic operation of a defibrillator to optimize a successful rescue outcome of a patient.

### SUMMARY OF THE INVENTION

The present disclosure improves upon a rescue protocol management of systematically operating a defibrillator between a scheduled operation mode and a custom operation mode by disabling an initial execution of the custom operation mode of the defibrillator preceding a delivery of an initial defibrillating shock to the heart of the patient and enabling the initial execution of the custom operation mode of the defibrillator succeeding the delivery of the initial defibrillating shock to the heart of the patient.

The present disclosure can be embodied as (1) a defibrillation controller, (2) a defibrillator, and (3) a defibrillation method.

Various embodiments of a defibrillation controller of the present disclosure employ a non-transitory machine-readable storage medium encoded with instructions for execution by one or more processors to systematically operate a defibrillator between a scheduled operation mode and a custom operation mode. The scheduled operation mode includes an uninterruptible CPR protocol of a fixed CPR time period and a scheduled shock protocol. The custom operation mode includes an interruptible CPR protocol of a terminal CPR time period and a custom shock protocol. An initial execution of the custom operation mode by the defibrillator is disabled by the defibrillation controller prior to a delivery of an initial defibrillating shock by the defibrillator to a heart of a patient, and the initial execution of the custom operation mode by the defibrillator is enabled by the defibrillation controller succeeding the delivery of the initial defibrillating shock by the defibrillator to the heart of the patient.

The defibrillation controller can be installed within and/or communicatively linked to an external defibrillator (e.g., various types of automatic external defibrillators, semi-automatic defibrillators and monitor/defibrillators).

Various embodiments of a defibrillator of the present disclosure employ a shock delivery circuit operable to deliver defibrillating shocks to a heart of a patient and further employs a defibrillation controller configured to systematically operate the defibrillator between a scheduled operation mode and a custom operation mode. The scheduled operation mode includes an uninterruptible CPR protocol of a fixed CPR time period and a scheduled shock protocol. The custom operation mode includes an interruptible CPR protocol of a terminal CPR time period and a custom shock protocol. An initial execution of the custom operation mode by the defibrillator is disabled by the defibrillation controller prior to a delivery of an initial defibrillating shock by the shock delivery circuit to a heart of a patient, and the initial execution of the custom operation mode by the defibrillator is enabled by the defibrillation controller succeeding the delivery of the initial defibrillating shock by the shock delivery circuit to the heart of the patient.

The defibrillator can be an external defibrillator (e.g., various types of automatic external defibrillators, semi-automatic defibrillators and monitor/defibrillators).

Various exemplary embodiments of a defibrillation method in accordance with the present disclosure involve systematically operating a defibrillator between a scheduled operation mode and a custom operation mode. The scheduled operation mode includes an uninterruptible CPR protocol of a fixed CPR time period and a scheduled shock protocol. The custom operation mode includes an interruptible CPR protocol of a terminal CPR time period and a custom shock protocol. An initial execution of the custom operation mode by the defibrillator is disabled by the defibrillation controller prior to a delivery of an initial defibrillating shock by the defibrillator to a heart of a patient, and the initial execution of the custom operation mode by the defibrillator is enabled by the defibrillation controller succeeding the delivery of the initial defibrillating shock by the defibrillator to the heart of the patient.

The defibrillation method can be implemented by a defibrillation controller installed within and/or communicatively linked to an external defibrillator (e.g., various types of automatic external defibrillators, semi-automatic defibrillators and monitor/defibrillators).

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following figures wherein:
FIG. 1 illustrates a cardiopulmonary resuscitation being administered by a responder or responders to a heart of a patient as known in the art of the present disclosure;
FIG. 2A illustrates a flowchart representative of a first exemplary embodiment of a strike-first rescue protocol of a defibrillation method in accordance with the present disclosure;
FIG. 2B illustrates a flowchart representative of a first exemplary embodiment of a CPR-first rescue protocol of a defibrillation method in accordance with the present disclosure;
FIGS. 3A-3D illustrate flowcharts representative of exemplary embodiments of a custom operation mode of a defibrillation method in accordance with the present disclosure;
FIG. 4A illustrates a flowchart representative of a shock-first operation mode of a defibrillation method in accordance with the present disclosure;
FIG. 4B illustrates a flowchart representative of a second exemplary embodiment of a shock-first rescue protocol of a defibrillation method in accordance with the present disclosure;
FIG. 4C illustrates a flowchart representative of a third exemplary embodiment of a shock-first rescue protocol of a defibrillation method in accordance with the present disclosure;
FIG. 5A illustrates a flowchart representative of a CPR-first operation mode of a defibrillation method in accordance with the present disclosure;
FIG. 5B illustrates a flowchart representative of a second exemplary embodiment of a CPR-first rescue protocol of a defibrillation method in accordance with the present disclosure;
FIG. 5C illustrates a flowchart representative of a third exemplary embodiment of a CPR-first rescue protocol of a defibrillation method in accordance with the present disclosure;
FIG. 6 illustrates an exemplary embodiment of a defibrillator in accordance with the present disclosure;
FIG. 7 illustrates an exemplary embodiment of a defibrillation controller in accordance with the present disclosure;
FIG. 8 illustrates an exemplary embodiment of an automatic/semi-automatic external defibrillator in accordance with the present disclosure; and
FIGS. 9A and 9B illustrates an exemplary embodiment of monitor/defibrillator in accordance with the present disclosure.

### DETAILED DESCRIPTION OF THE PREFERRED EMBODIMENTS

The present disclosure is directed to disabling an initial execution of a custom operation mode of a defibrillator preceding an initial delivery of a defibrillating shock to a heart of a patient during a resuscitation rescue effort of the patient by a responder or responders, and enabling the initial execution of the custom operation mode of a defibrillator succeeding the initial delivery of the defibrillating shock to the heart of the patient during the resuscitation rescue effort of the patient by the responder(s).

For purposes of describing and claiming the present disclosure,
(1) terms of the art of the present disclosure, but not limited to, "defibrillation", "defibrillator", "defibrillating shock", "electrocardiogram (ECG)", "cardiac rhythm", "cardiopulmonary resuscitation (CPR)", "rescue protocol", "CPR protocol", "shock protocol", "scheduled operation mode" and "custom operation mode" are to be interpreted as known in the art of the present disclosure and as exemplary described in the present disclosure;
(2) the term "C-Shock Advisory" broadly encompasses all methods, as known in the art of the present disclosure or hereinafter conceived, for analyzing and classifying a cardiac rhythm of an ECG of a heart of a patient including artifacts as known in the art of the present resulting from an administration of chest compressions to the heart of the patient (i.e., a corrupt ECG) to thereby derive
   (a) a shock decision based upon a determinate classification of the corrupt ECG as having a shockable cardiac rhythm (e.g., a ventricular fibrillation (VF) rhythm or a ventricular tachycardia (VT) rhythm),
   (b) a non-shock decision based upon a determinate classification of the corrupt ECG as having a non-shockable cardiac rhythm (e.g., a pulseless electrical activity rhythm or an asystole rhythm), or
   (c) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(3) the term "C-Shock Delivery" broadly encompasses all methods, as known in the art of the present disclosure or hereinafter conceived, for delivering a defibrillating shock to a heart of a patient corresponding to a shock decision derived by the C-Shock Advisory in accordance with rule(s)/guideline(s) associated with a rescue protocol (e.g., rule(s)/guideline(s) established by the American Heart Association);
(4) the term "F-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from a termination/suspension of an administration of chest compressions to the heart of the patient (i.e., a clean ECG) to thereby derive
   (a) a shock decision based upon a determinate classification of the clean ECG as having a shockable cardiac rhythm (e.g., a ventricular fibrillation (VF) rhythm or a ventricular tachycardia (VT) rhythm),
   (b) a non-shock decision based upon a determinate classification of the clean ECG as having a non-shockable cardiac rhythm (e.g., a pulseless electrical activity rhythm or an asystole rhythm, or
   (c) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(5) the term "F-Shock Delivery" broadly encompasses all methods, as known in the art of the present disclosure or hereinafter conceived, for delivering a defibrillating shock to a heart of a patient corresponding to a shock decision derived by the F-Shock Advisory in accordance with rule(s)/guideline(s) associated with a rescue protocol (e.g., rule(s)/guideline(s) established by the American Heart Association); and
(6) the term "CPR Quality Analysis" broadly encompasses all methods, as known in the art of the present disclosure or hereinafter conceived, for monitoring a quality of CPR being administered to a heart of a patient to derive an assessment of a high quality/compliant CPR or a low quality/non-compliant CPR.

A non-limiting example of a C-Shock Advisory is an Arrythmia Recognition Technology (ART) as known in the art of the present disclosure.

A non-limiting example a F-Shock Advisory is a Patient Analysis System (PAS) as known in the art of the present disclosure.

In practice, for the C-Shock Advisory and the F-Shock Advisory, one or more of the described shock decisions can be omitted and/or additional shock decision(s) can be derived from an analysis and rhythm classification of ECG.

A non-limiting example of CPR Quality Analysis is a monitoring of a depth of compression, a rate of compression and chest release and recoil effective relative to rule(s)/guideline(s) of a rescue protocol (e.g., rule(s)/guideline(s) established by the American Heart Association) to derive an assessment of a high quality/compliant CPR being administered to the heart of the patient or a low quality/non-compliant CPR being administered to the heart of the patient.

An additional non-limiting example of CPR Quality Analysis is an implementation of a physiological sensor/monitor for measuring blood flow, cerebral perfusion and/or myocardial perfusion.

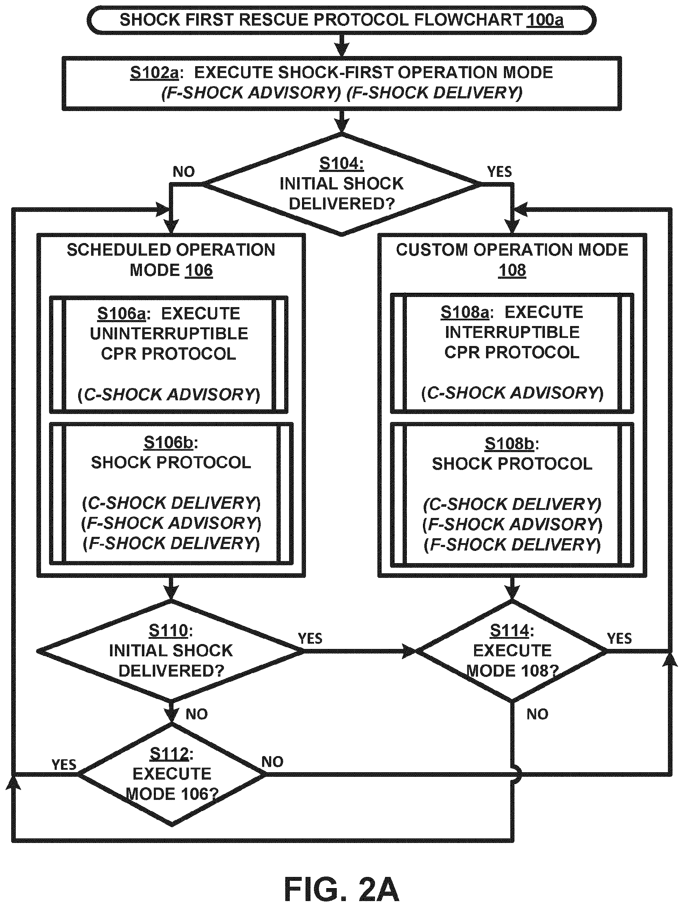

To facilitate an understanding of the present disclosure, the following description of FIGS. 2A-5C describes and teaches exemplary embodiments of methods in accordance with the present disclosure. From the description of FIGS. 2A-5C, those having ordinary skill in the art of the present disclosure will appreciate how to apply the present disclosure to make and use additional embodiments of methods in accordance with the present disclosure.

FIG. 2A illustrates a shock-first rescue protocol flowchart 100a of the present disclosure. Referring to FIG. 2A, a stage S 102a of flowchart 100 encompasses an execution of a shock-first operation mode including (1) a F-Shock Advisory of a "Shock" decision, or a "No Shock/Undecided" decision dependent upon a classification of a clean ECG as a shockable cardiac rhythm, a non-shockable cardiac rhythm, or an organized cardiac rhythm, respectively, and (2) a F-Shock Delivery of an initial defibrillating shock if the F-Shock Advisory was a "Shock" decision.

Flowchart 100a proceeds to a stage S 104 to determine if an initial defibrillating shock was delivered via F-Shock Delivery during stage S 102a. Flowchart 100a proceeds to a scheduled operation mode 106 if stage S 104 determines an initial defibrillating shock was not delivered during stage S 102a, or otherwise proceeds to a custom operation mode 108 if stage S 104 determines an initial defibrillating shock was delivered during stage S 102a.

Still referring to FIG. 2A, scheduled operation mode 106 includes (1) a stage S 106a encompassing an execution of an uninterruptible CPR protocol having a fixed CPR time period (e.g., two (2) minutes) and (2) a stage S 106b encompassing an execution of a shock protocol.

For this embodiment, stage S 106a encompasses (1) a communication of CPR administration instructions to a responder (not shown) or a CPR mechanical device (not shown) for administering CPR to the heart of the patient, and (2) a C-Shock Advisory of a "Shock" decision or a "No Shock/Undecided" decision dependent upon a classification of a corrupt ECG as a shockable cardiac rhythm, a non-shockable cardiac rhythm, or an organized cardiac rhythm, respectively.

Flowchart 100a proceeds to stage S 106b, which encompasses (1) a communication of CPR termination instructions to a responder (not shown) or a CPR mechanical device (not shown) for terminating an administration of CPR to the heart of the patient, and (2) a delivery of a defibrillating shock if the C-Shock Advisory of stage S 106a was a "Shock" decision upon termination of the fixed CPR time period of stage S 106a.

If the C-Shock Advisory of stage S 106a was a `No Shock/Undecided" decision, then Stage S106b further encompasses (1) a F-Shock Advisory of a "Shock" decision or a "No Shock/Undecided" decision dependent upon a classification of a clean ECG as a shockable cardiac rhythm, a non-shockable cardiac rhythm, or an organized cardiac rhythm, respectively, and (2) a F-Shock Delivery of a defibrillating shock if the F-Shock Advisory was a "Shock" decision.

Still referring to FIG. 2A, custom operation mode 108 includes (1) a stage S108a encompassing an execution of an interruptible CPR protocol having a terminal CPRtime period (e.g., a maximum time of two (2) minutes that can be pre-timely terminated or timely terminated), and (2) a stage S 108b encompassing an execution of a shock protocol.

For this embodiment, stage S 108a encompasses (1) a communication of CPR administration instructions to a responder (not shown) or a CPR mechanical device (not shown) for administering CPR to the heart of the patient, and (2) a C-Shock Advisory of a "Shock" decision, or a "No Shock/Undecided" decision dependent upon a classification of a corrupt ECG as a shockable cardiac rhythm, a non-shockable cardiac rhythm, or an organized cardiac rhythm, respectively.

Flowchart 100a pre-timely terminates the terminal CPRtime period of stage S 108a and proceeds to stage S 108b if the C-Shock Advisory of stage S 108a is a "Shock" decision, or timely terminates the terminal time period of stage S108a and proceeds to stage S 108b if the C-Shock Advisory of stage S 108a is a "No Shock/Undecided" decision.

Stage S 108b encompasses (1) a communication of CPR termination instructions to a responder (not shown) or a CPR mechanical device (not shown) for terminating an administration of CPR to the heart of the patient, and (2) a delivery of a defibrillating shock if the C-Shock Advisory of stage S 108a was a "Shock" decision.

Conversely, if the C-Shock Advisory of stage S 108a was a `No Shock/Undecided" decision, then stage S 108b further encompasses (1) a F-Shock Advisory of a "Shock" decision, or a "No Shock/Undecided" decision dependent upon a classification of a clean ECG as a shockable cardiac rhythm, a non-shockable cardiac rhythm, or an organized cardiac rhythm, respectively and (2) a F-Shock Delivery of a defibrillating shock if the F-Shock Advisory was a "Shock" decision.

Still referring to FIG. 2A, those having ordinary skill in the art will appreciate stage S 104 of flowchart 100a disables an initial execution of custom operation mode 108 preceding an initial shock delivery to the heart of the patient, and conversely enables an initial execution of custom operation mode 108 succeeding an initial shock delivery to the heart of the patient.

Furthermore, upon a completed execution of stage S 106, a stage 110 of flowchart 100a encompasses a determination if an initial defibrillating shock was delivered to the heart of the patient during the initial execution of scheduled operation mode 106. Flowchart 100a proceeds to a stage S 112 if an initial defibrillating shock was not delivered during the completed execution of stage S 106 or otherwise proceeds to a S 114 of flowchart 100a if an initial defibrillating shock was delivered to the heart of the patient during the initial execution of stage S 106.

Stage S 112 encompasses a determination of whether or not flowchart 100a should return to scheduled operation mode 106 or proceed to custom operation mode 108. Flowchart 100a will automatically return to scheduled operation mode 106 if an initial defibrillating shock to the heart of the patient was not delivered during the initial execution of scheduled operation mode 106 nor any succeeding executions of scheduled operation mode 106. Otherwise, if the initial defibrillating shock to the heart of the patient was delivered during the initial execution of scheduled operation mode 106 or any succeeding execution of scheduled operation mode 106, then flowchart 100a will return to scheduled operation mode 106 or proceed to custom operation mode 108 dependent upon rules/guidelines specifying a preference between scheduled operation mode 106 and custom operation mode 108 if view of operational factor(s) upon the preceding execution of schedule operation mode 106 (e.g., the total number of shock deliveries to the heart of the patient).

Stage S 114 encompasses a determination of whether or not flowchart 100a should proceed/return to custom operation mode 108 or proceed/return to scheduled operation mode 106. Flowchart 100a will automatically proceed to custom operation mode 108 if an initial defibrillating shock was delivered during the preceding execution of scheduled operation mode 106 as determined during stage S 110. Otherwise, 108 if an initial defibrillating shock was delivered during the preceding execution of scheduled operation mode 106 as determined during stage S110, then flowchart 100a will return to scheduled operation mode 106 or return to custom operation mode 108 dependent upon rules/guidelines specifying a preference between scheduled operation mode 106 and custom operation mode 108 upon the preceding execution of custom operation mode 108 (e.g., the total number of shock deliveries to the heart of the patient).

Still referring to FIG. 2A, those having ordinary skill in the art will appreciate stage S110 of flowchart 100a disables an initial execution of custom operation mode 108 prior to a delivery of an initial defibrillating shock to a heart of a patient by scheduled operation mode 106 if the initial defibrillating shock was not delivered during stage S 102a, and enables the initial execution of custom operation mode 108 subsequent to a delivery of an initial defibrillating shock to a heart of a patient by scheduled operation mode 106 if the initial defibrillating shock was not delivered during stage S102a.

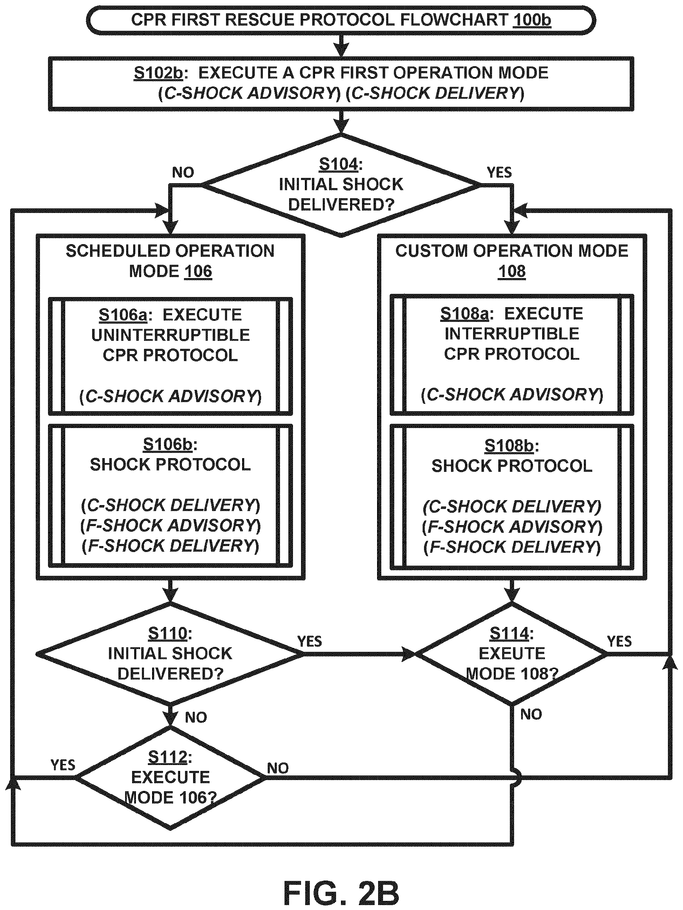

FIG. 2B illustrates a CPR-first rescue protocol flowchart 100b of the present disclosure. Alternative to stage S102a of shock-first rescue protocol flowchart 100a (FIG. 2A), referring to FIG. 2B, a stage S102b of flowchart 100b encompasses (1) a communication of CPR administration instructions to a responder for a fixed CPR time period (not shown) (e.g., chest compression only or chest compression and breaths), (2) a C-Shock Advisory of a "Shock" decision or a "No Shock/Undecided" decision dependent upon a classification of a corrupt ECG as a shockable cardiac rhythm, a non-shockable cardiac rhythm, or an organized cardiac rhythm, respectively, and (3) a C-Shock Delivery of an initial defibrillating shock if the C-Shock Advisory was a "Shock" decision upon a timely termination of the fixed CPR time period.

Still referring to FIG. 2B, stages S104-S114 of flowchart 100b are identical to stages S104-114 of flowchart 100a (FIG. 2A). Again, those having ordinary skill in the art will appreciate stages S104 and S110 of flowchart 100b disable an initial execution of custom operation mode 108 prior to a delivery of an initial defibrillating shock to a heart of a patient by stage S102b or scheduled operation mode 106, and enables an initial execution of custom operation mode 108 subsequent to a delivery of an initial defibrillating shock to a heart of a patient by stage S102a or scheduled operation mode 106.

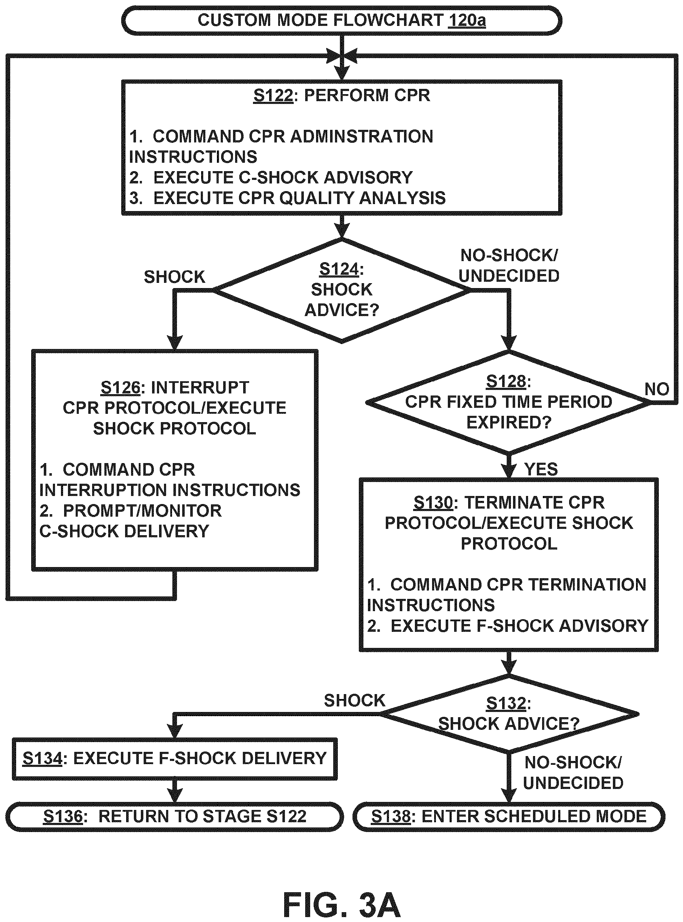

FIGS. 3A-3D illustrate various embodiments of custom operation mode 108 and stage S 114 of FIGS. 2A and 2B.

FIG. 3A illustrates a base custom operation mode flowchart 120a of the present disclosure. Referring to FIG. 3A, a stage S122 of flowchart 120 encompasses (1) a communication of CPR administration instructions to a responder (not shown) or a CPR mechanical device (not shown) for administering CPR to the heart of the patient, (2) a C-Shock Advisory of a "Shock" decision or a "No Shock/Undecided" decision dependent upon a classification of a corrupt ECG as a shockable cardiac rhythm, a non-shockable cardiac rhythm, or an organized cardiac rhythm, respectively and (3) an optional monitoring of CPR Quality Analysis of the CPR being administered by the responder or the CPR mechanical device.

A stage S 124 of flowchart 120a determines if the C-Shock Advisory of stage S 122 is "Shock" decision or "No Shock/Undecided" decision. If the C-Shock Advisory is a "Shock" decision for stage S 124, then flowchart 120a proceeds to a stage S 126 to interrupt the CPR protocol and execute the shock protocol including (1) issuing CPR interruption instructions to the responder (not shown) or CPR mechanical device (not shown), and (2) prompting and monitoring a C-Shock Delivery of the defibrillation shock as known in the art of the present disclosure.

If the C-Shock Advisory is a "No Shock/Undecided" decision for stage S 124, then flowchart 120a proceeds to a stage S 128 to determine if a CPR fixed CPR time period has timely expired (e.g., two minutes). If the CPR fixed CPR time period has not timely expired, then flowchart 120a returns to stage S 122. If the CPR fixed CPR time period has timely expired, then flowchart 120a proceeds to a stage S 130 of flowchart S 102a to terminate the CPR protocol and execute the shock protocol including (1) a communication of CPR termination instructions to a responder (not shown) or a CPR mechanical device (not shown) and (2) a F-Shock Advisory of a "Shock" decision or a "No Shock/Undecided" decision dependent upon a classification of a clean ECG as a shockable cardiac rhythm, a non-shockable cardiac rhythm, or an organized cardiac rhythm, respectively.

A stage S 132 of flowchart 120a determines if the F-Shock Advisory of stage S130 is "Shock" decision or "No Shock/Undecided" decision.

If the F-Shock Advisory is "Shock" for stage S130, then flowchart 120a proceeds to a stage S 134, which encompasses a prompting and monitoring of a F-Shock Delivery to the heart of the patient as known in the art of the present disclosure. A stage S 136 of flowchart 120a thereafter returns to stage S 122.

If the F-Shock Advisory is a "No Shock/Undecided" decision for stage S130, then a stage S138 of flowchart 120a proceeds to scheduled operation mode 106 (FIGS. 2A and 2B).

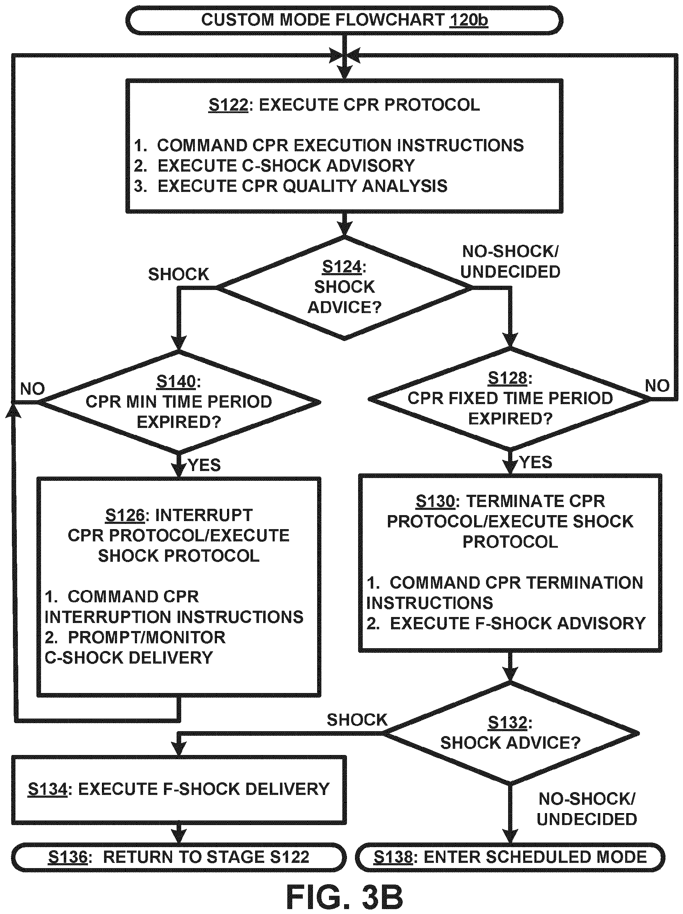

FIG. 3B illustrates a time based custom operation mode flowchart 120b of the present disclosure. Referring to FIG. 3B, flowchart 120b is a modified version of flowchart 120a (FIG. 3A) incorporating a stage S 140 to determine if a CPR minimum time period has timely expired upon a C-Shock Advisory of a "Shock" decision during stage S124.

If stage S 140 determines the minimum time period has not timely expired, then flowchart 120b returns to stage S 122 as previously described in the present disclosure. Otherwise, if stage S 140 determines the minimum time period of stage S 140 has timely expired, then flowchart 120b proceeds to stage S 126 as previously described in the present disclosure.

In practice, the minimum time period can be fixed or configurable (e.g., by the responder).

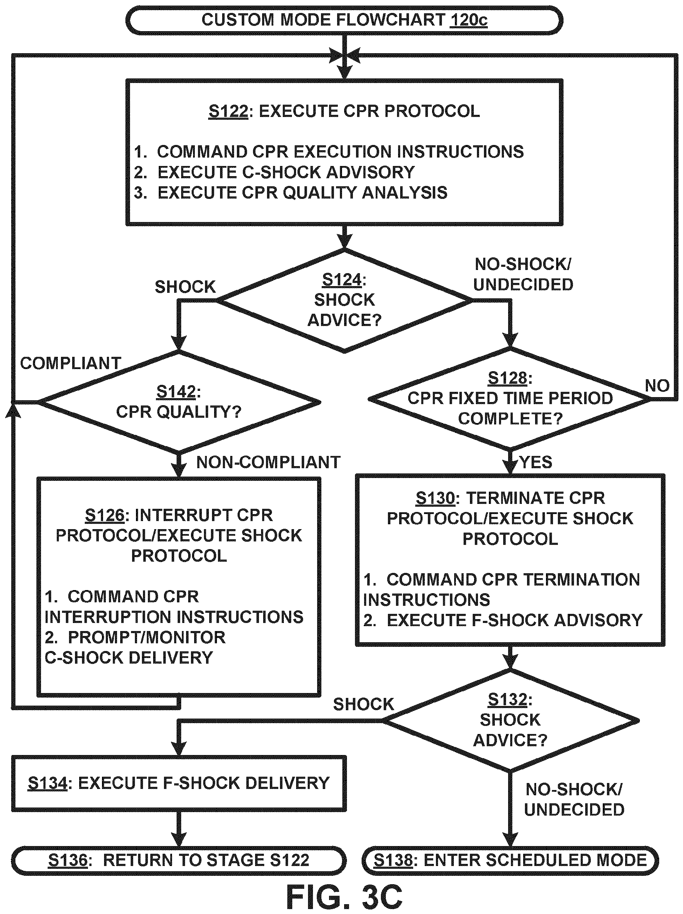

FIG. 3C illustrates a CPR quality based custom operation mode flowchart 120c of the present disclosure. Referring to FIG. 3C, flowchart 120c is a modified version of flowchart 120a (FIG. 3A) incorporating a stage S142 encompassing results of the CPR Quality Analysis of stage S 122 upon a C-Shock Advisory of a "Shock" decision during stage S 122 as previously described in the present disclosure.

If the CPR Quality Analysis is a "Compliant" decision, then flowchart 120c returns to stage S 122. Otherwise, the CPR Quality Analysis is a "Non-Compliant" decision, then flowchart 120c proceeds to stage S 126 as previously described in the present disclosure.

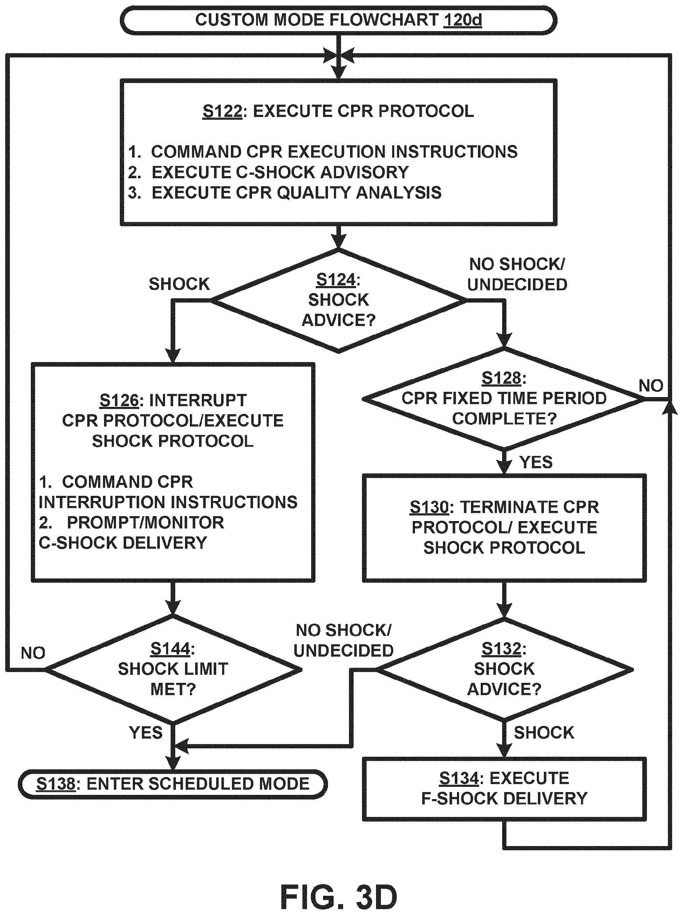

FIG. 3D illustrates a shock limit based custom operation mode flowchart 120d of the present disclosure. Referring to FIG. 3D, flowchart 120d is a modified version of flowchart 120a (FIG. 3A) incorporating a stage S 144 to determine if a maximum number of defibrillation shocks of stage S 126 have been delivered to the heart of the patient.

If the maximum number of defibrillating shocks of stage S 126 have not been delivered to the heart of the patient, then flowchart 120d returns to stage S 122. Otherwise, if the maximum number of defibrillating shocks of stage S 126 have not been delivered to the heart of the patient, then flowchart 120d proceeds to stage S138 to enter scheduled operation mode 106 (FIG. 2A and 2B).

The preceding description of FIGS. 2A-3C were directed to fundamental rhythm classifications of an ECG as a basis for a conditional delivery of a defibrillating shock to a heart of a patient.

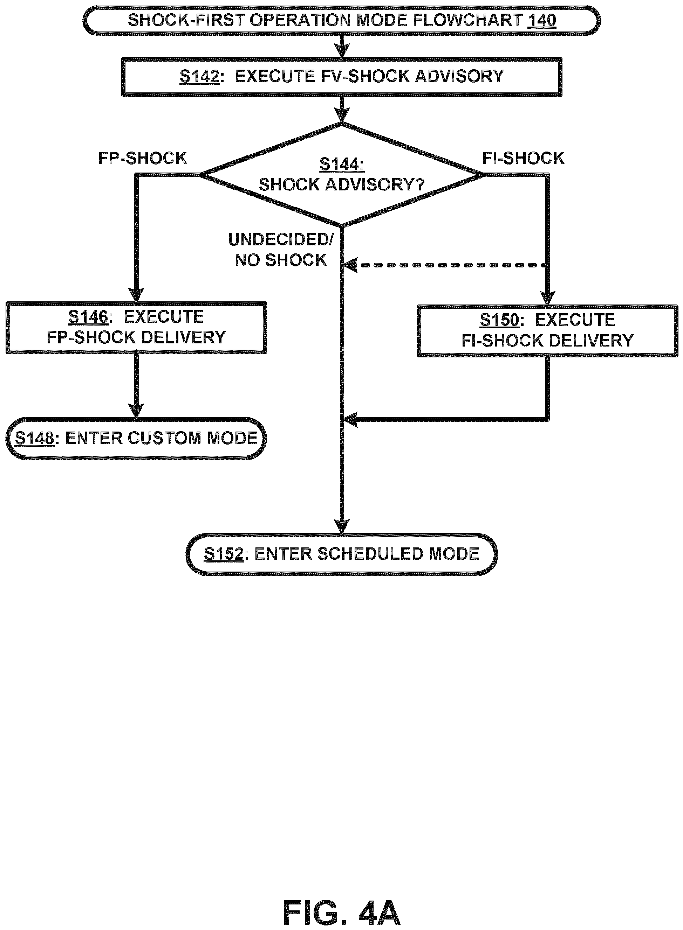

The succeeding description of FIGS. 4A-5C are directed to an enhanced rhythm classifications of an ECG as a basis for a conditional delivery of a defibrillating shock to a heart of a patient.

For purposes of describing and claiming the present disclosure,
(1) the term "rescue outcome" broadly encompasses a defibrillation of a heart of a patient with or without resuscitation of the patient (e.g., a return of an organized cardiac rhythm with or without a sustained return of spontaneous circulation) and further broadly encompasses a resuscitation of the patient (e.g., a defibrillation of the heart with a sustained return of spontaneous circulation or a survival of the patient with a cerebral performance category 1 or a cerebral performance category 2);
(2) the term "probable rescue outcome" broadly encompasses a probability of a defibrillation of a heart derived, directly or indirectly, from a successful restoration of an organized rhythm and contractile function of a heart from a delivery of a defibrillating shock to the heart of the patient;
(3) the term "improbable rescue outcome" broadly encompasses an improbability of a defibrillation of the heart derived, directly or indirectly, from an unsuccessful restoration of an organized rhythm and contractile function of the heart from a delivery of a defibrillation shock to the heart of the patient;
(4) the term "probable defibrillating shock" broadly encompasses a defibrillating shock delineated as having a probable rescue outcome;
(5) the term "improbable defibrillating shock" broadly encompasses a defibrillating shock delineated as having an improbable rescue outcome;
(6) the term "CV-Shock Advisory" broadly encompasses all methods, as known in the art of the present disclosure or hereinafter conceived, for analyzing and classifying a cardiac rhythm of an ECG of a heart of a patient including artifacts as known in the art of the present resulting from an administration of chest compressions to the heart of the patient (i.e., a corrupt ECG) and for predicting a probable rescue outcome or an improbable rescue outcome to thereby derive
   (a) a CP-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of a probable rescue outcome,
   (b) a CI-shock decision based upon a determinate classification of the corrupt ECG as having a shockable cardiac rhythm (e.g., a ventricular fibrillation (VF) rhythm or a ventricular tachycardia (VT) rhythm) and based further upon a prediction of an improbable rescue outcome,
   (c) a non-shock decision based upon a determinate classification of the corrupt ECG as having a non-shockable cardiac rhythm (e.g., a pulseless electrical activity rhythm or an asystole rhythm, or
   (d) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(7) the term "CP-Shock Delivery" broadly encompasses all methods, as known in the art of the present disclosure or hereinafter conceived, for delivering a defibrillating shock to a heart of a patient corresponding to a CP-shock decision derived by the CV-Shock Advisory in accordance with rule(s)/guideline(s) associated with a rescue protocol (e.g., rule(s)/guideline(s) established by the American Heart Association);
(8) the term "CI-Shock Delivery" broadly encompasses all methods, as known in the art of the present disclosure or hereinafter conceived, for delivering a defibrillating shock to a heart of a patient corresponding to a CI-shock decision derived by the CV-Shock Advisory in accordance with rule(s)/guideline(s) associated with a rescue (e.g., rule(s)/guideline(s) established by the American Heart Association);
(9) the term "FV-Shock Advisory" broadly encompasses all methods, as known in the art of the present disclosure or hereinafter conceived, for analyzing and classifying a cardiac rhythm of an ECG of a heart of a patient excluding artifacts as known in the art of the present disclosure resulting from an administration of chest compressions to the heart of the patient (i.e., a clean ECG) and for predicting a probable rescue outcome or an improbable rescue outcome to thereby derive
   (a) a FP-shock decision based upon a determinate classification of the clean ECG as having a shockable cardiac rhythm (e.g., a ventricular fibrillation (VF) rhythm or a ventricular tachycardia (VT) rhythm) and upon a prediction of a probable rescue outcome,
   (b) a FI-shock decision based upon a determinate classification of the clean ECG as having a shockable cardiac rhythm (e.g., a ventricular fibrillation (VF) rhythm or a ventricular tachycardia (VT) rhythm) and upon a prediction of an improbable rescue outcome,
   (c) a non-shock decision based upon a determinate classification of the clean ECG as having a non-shockable cardiac rhythm (e.g., a pulseless electrical activity rhythm or an asystole rhythm, or
   (d) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm);
(10) the term "FP-Shock Delivery" broadly encompasses all methods, as known in the art of the present disclosure or hereinafter conceived, for delivering a defibrillating shock to a heart of a patient corresponding to a FP-shock decision derived by the FV-Shock Advisory in accordance with rule(s)/guideline(s) associated with a rescue protocol (e.g., rule(s)/guideline(s) established by the American Heart Association); and
(11) the term "FI-Shock Delivery" broadly encompasses all methods, as known in the art of the present disclosure or hereinafter conceived, for delivering a defibrillating shock to a heart of a patient corresponding to a FI-shock decision derived by the FV-Shock Advisory in accordance with rule(s)/guideline(s) associated with a rescue protocol (e.g., rule(s)/guideline(s) established by the American Heart Association).

A non-limiting example of a FV-Shock Advisory is a Patient Analysis System (PAS) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

A non-limiting example of a CV-Shock Advisory is an Arrythmia Recognition Technology (ART) as known in the art of the present disclosure incorporating a measurement of a vitality of a shockable cardiac rhythm as known in the art of the present disclosure (e.g., a measurement of an amplitude/frequency of the shockable cardiac rhythm relative to a sensitivity/specificity based threshold delineating between a probable rescue outcome or an improbable rescue outcome).

In practice, for the CV-Shock Advisory and the FV-Shock Advisory, one or more of the described shock decisions can be omitted and/or additional shock decision(s) can be derived from an analysis and classification of a cardiac rhythm of an ECG.

FIG. 4A illustrates a shock-first operation mode flowchart 140 of the present disclosure. Referring to FIG. 4A, a stage S142 of flowchart 140 encompasses an execution of a FV-Shock Advisory. A stage S144 of flowchart 140 proceeds to a stage S146 to execute a FP-Shock Delivery and a stage S148 to enter the custom operation mode if the FV-Shock Advisory of stage S142 is a "FP-Shock" decision, or proceeds to a stage S150 to execute a FI-Shock Delivery and a stage S152 to enter the scheduled operation mode if the FV-Shock Advisory of stage S142 is a "FI-Shock" decision, or proceeds to stage S150 to enter the scheduled operation mode if the FV-Shock Advisory of stage S142 is a "No Shock/Undecided" decision.

In alterative embodiment, as symbolized by the dashed arrow, flowchart 140 proceeds from stage S142 to stage S152 to enter the scheduled operation mode if the FV-Shock Advisory of stage S142 is a "FI-Shock" decision.

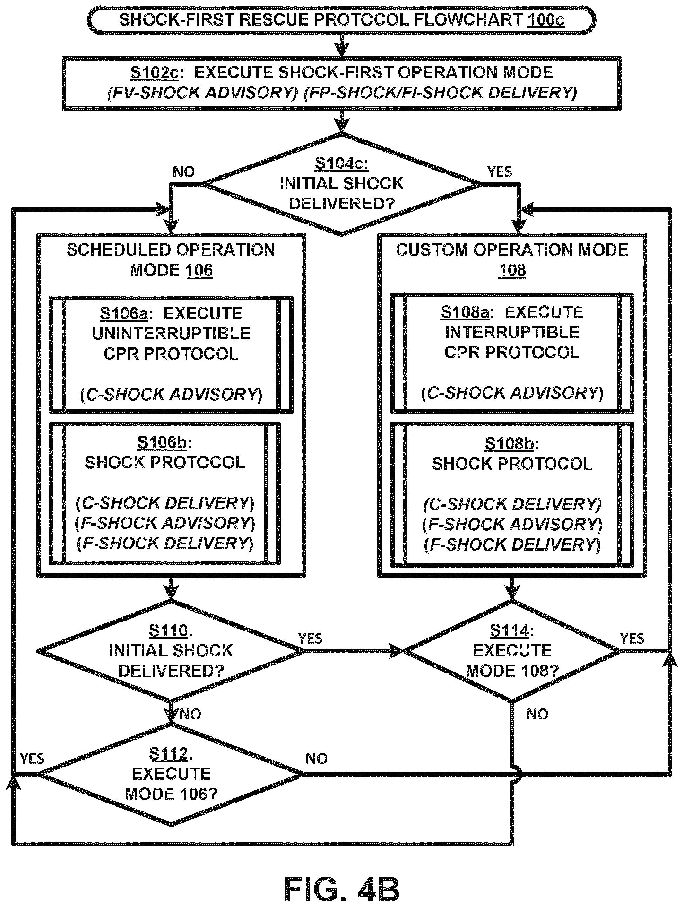

FIG. 4B illustrates a shock-first rescue protocol flowchart 100c. Referring to FIG. 4B, flowchart 100c is a modified version of flowchart 100a (FIG. 2A) replacing stage S102a with a stage S102c incorporating the shock-first operation mode of FIG. 4A. Specifically, a stage S104c of flowchart 100c proceeds to scheduled operation mode 106 if the FV-Shock Advisory of stage S142 determines a "FI-Shock" decision or "No Shock/Undecided" decision as shown in FIG. 4A.

Otherwise, stage S104c of flowchart 100c proceeds to custom operation mode 108 if the FV-Shock Advisory of stage S142 determines a "FP-Shock" decision as shown in FIG. 4A.

Still referring to FIG. 4B, those having ordinary skill in the art of the present disclosure will appreciate stage S104c of flowchart 100c disables an initial execution of custom operation mode 108 preceding an initial vitality shock delivery to the heart of the patient, and conversely enables an initial execution of custom operation mode 108 succeeding an initial vitality shock delivery to the heart of the patient.

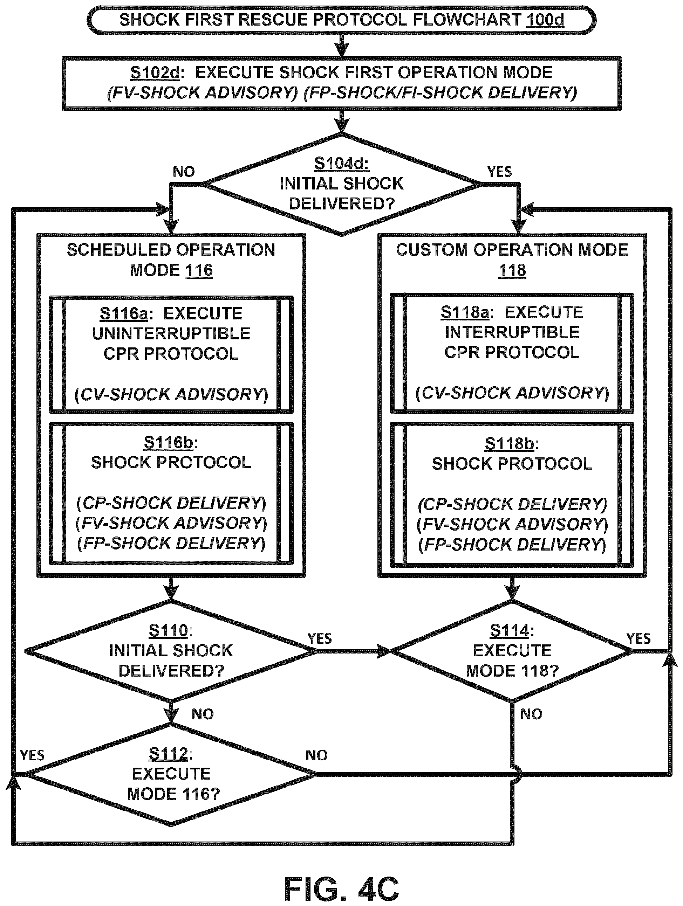

FIG. 4C illustrates a shock-first rescue protocol flowchart 100d. Referring to FIG. 4C, flowchart 100d is a modified version of flowchart 100c (FIG. 4B) with a stage S102d as a duplication of stage S102c (FIG. 4B) and replacing scheduled operation mode 106 (FIGS. 2A and 2B) with a scheduled operation mode 116 incorporating a CV-Shock Advisory, a CP-Shock Delivery, a FV-Shock Advisory, and a FP-Shock Delivery. Flowchart 100d also replaces custom operation mode 108 (FIGS. 2A and 2B) with a custom operation mode 118 incorporating a CV-Shock Advisory, a CP-Shock Delivery, a FV-Shock Advisory, and a FP-Shock Delivery.

Those having ordinary skill in the art of the present disclosure will appreciate stage S104d of flowchart 100d disables an initial execution of custom operation mode 118 preceding an initial vitality shock delivery to the heart of the patient, and conversely enables an initial execution of custom operation mode 118 succeeding an initial vitality shock delivery to the heart of the patient.

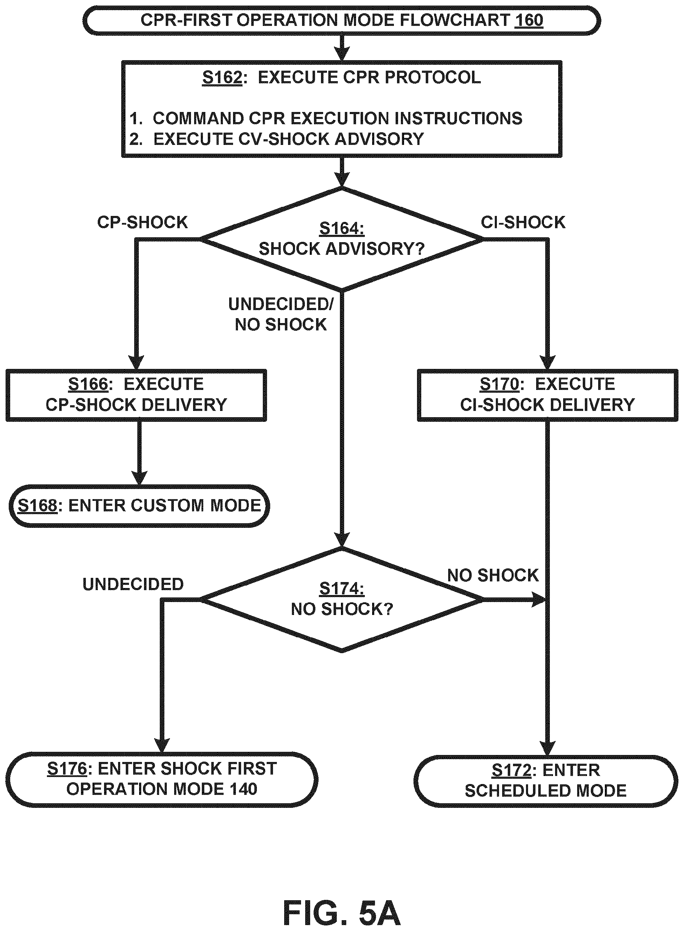

FIG. 5A illustrates a CPR-first operation mode flowchart 160 of the present disclosure. Referring to FIG. 5A, a stage S162 of flowchart 160 encompasses an execution of a CPR protocol including issuing CPR execution instruction(s) as needed, and an execution of a CV-Shock Advisory. A stage S164 of flowchart 160 proceeds to a stage S166 to execute a CP-Shock Delivery and a stage S168 to enter the custom operation mode if the CV-Shock Advisory of stage S162 is a "CP-Shock" decision, or proceeds to a stage S170 to execute a CI-Shock Delivery and a stage S172 to enter the scheduled operation mode if the CV-Shock Advisory of stage S162 is a "CI-Shock" decision, or proceeds to stage S172 to enter the scheduled operation mode if the CV-Shock Advisory of stage S162 is a "No Shock/Undecided" decision.

From stage S174, flowchart 160 proceeds to stage S172 to enter the scheduled operation mode if the CV-Shock Advisory of stage S162 is a "No Shock" decision, or proceeds to stage S176 to enter the shock-first operation mode 140 if the CV-Shock Advisory of stage S162 is a "Undecided" decision.

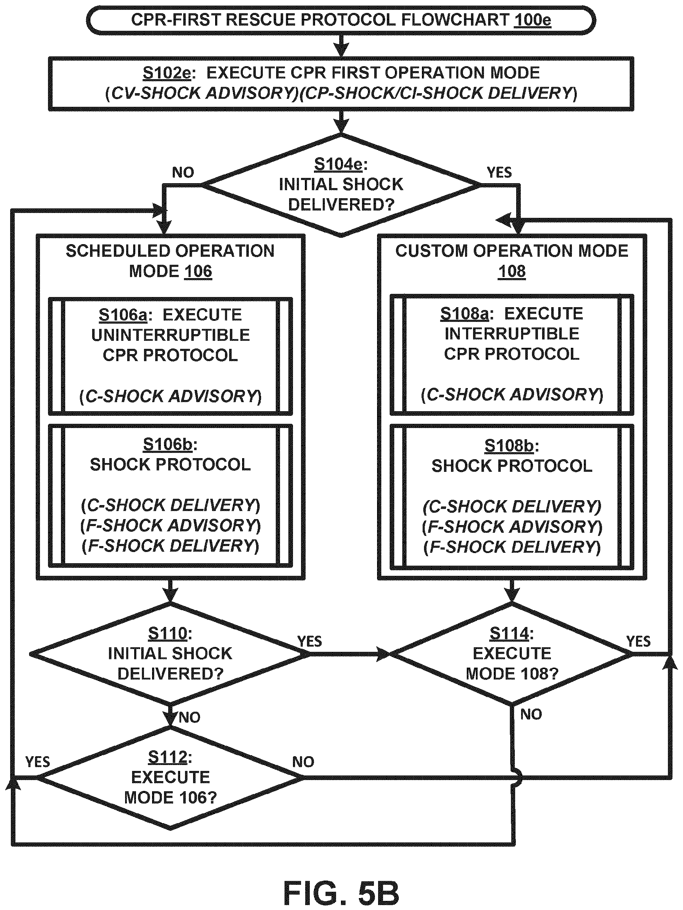

FIG. 5B illustrates a CPR-first rescue protocol flowchart 100e. Referring to FIG. 5B, flowchart 100e is a modified version of flowchart 100a (FIG. 2A) replacing stage S102a with a stage S102e incorporating the CPR-first operation mode of FIG. 5A. Specifically, a stage S104e of flowchart 100e will proceed to scheduled operation mode 106 if the CV-Shock Advisory of stage S162 (FIG. 5A) determines a "CI-Shock" decision or a "No Shock" decision, or proceeds to custom operation mode 108 if the CV-Shock Advisory of stage S162 (FIG. 5A) determines a "CP-Shock" decision, or proceeds to flowchart 140 if the CV-Shock Advisory of stage S162 (FIG. 5A) determines a "Undecided" decision.

Still referring to FIG. 5B, those having ordinary skill in the art of the present disclosure will appreciate stage S104e of flowchart 100e disables an initial execution of custom operation mode 108 preceding an initial vitality shock delivery to the heart of the patient, and conversely enables an initial execution of custom operation mode 108 succeeding an initial vitality shock delivery to the heart of the patient.

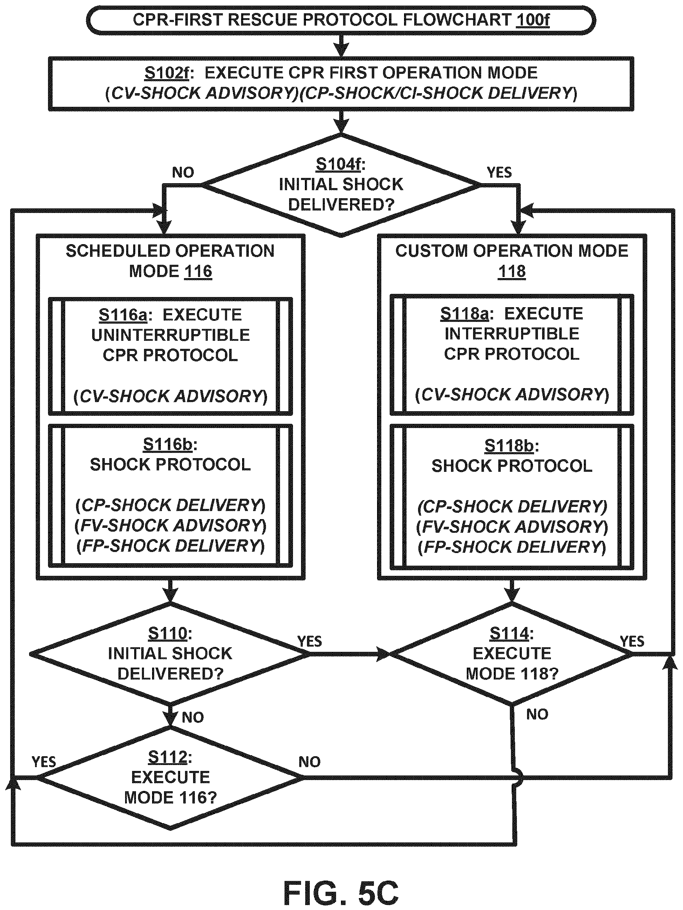

FIG. 5C illustrates a CPR-first rescue protocol flowchart 100f. Referring to FIG. 5C, flowchart 100f is a modified version of flowchart 100e (FIG. 5B) ) with a stage S102f as a duplication of stage S102e (FIG.5B) and replacing scheduled operation mode 106 (FIGS. 2A and 2B) with a scheduled operation mode 116 incorporating a CV-Shock Advisory, a CP-Shock Delivery, a CV-Shock Advisory, and a CP-Shock Delivery. Flowchart 100f also replaces custom operation mode 108 (FIGS. 2A and 2B) with a custom operation mode 118 incorporating a CV-Shock Advisory, a CP-Shock Delivery, a CV-Shock Advisory, and a CP-Shock Delivery.

Those having ordinary skill in the art of the present disclosure will appreciate stage S104f of flowchart 100f disables an initial execution of custom operation mode 118 preceding an initial vitality shock delivery to the heart of the patient, and conversely enables an initial execution of custom operation mode 118 succeeding an initial vitality shock delivery to the heart of the patient.

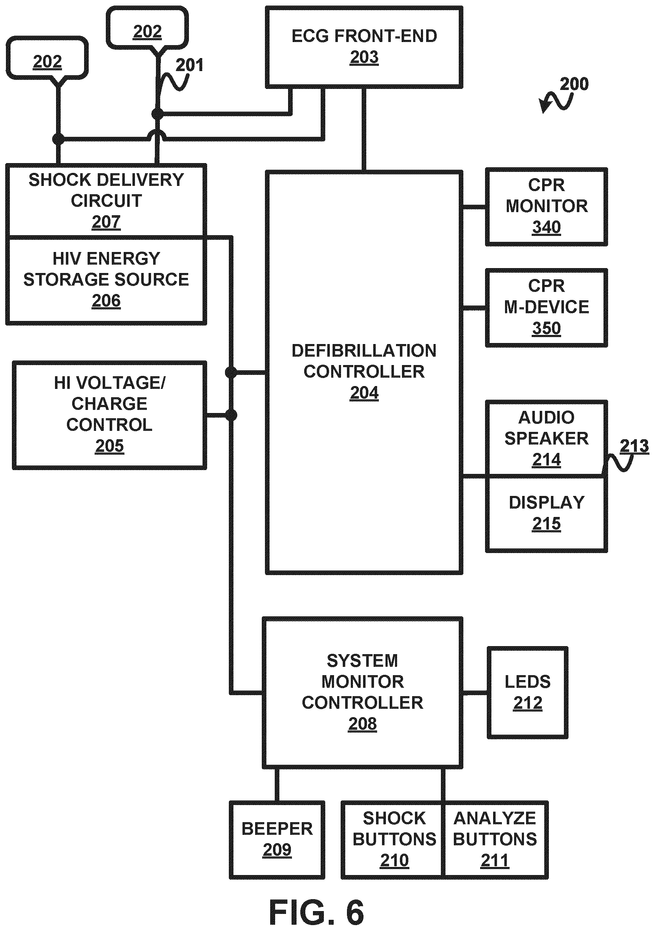

To facilitate a further understanding of the present disclosure, the following description of FIGS. 6-9B teaches exemplary embodiments of devices and systems in accordance with the present disclosure. From the description of FIGS. 6-9B, those having ordinary skill in the art of the present disclosure will appreciate how to apply the present disclosure to make and use additional embodiments of devices and systems in accordance with the present disclosure.

The afore-described methods can be implemented in a medical device, such as, for example, a defibrillator (e.g., internal, external or manual). FIG. 6 is a functional block diagram of an external defibrillator 204 according to the one embodiment of the present disclosure. Defibrillator 204 is configured as an AED that is intended for use during a cardiac rescue which includes CPR. It is designed for small physical size, light weight, and relatively simple user interface capable of being operated by personnel without high training levels or who otherwise would use the defibrillator 204 only infrequently. Although the present embodiment of the invention is described with respect to application in an AED, other embodiments include application in different types of defibrillators, for example, manual defibrillators, fully automatic defibrillators, and paramedic or clinical defibrillator/monitors.

Defibrillator 204 receives an input 214 of an ECG signal from, for example, two or more electrodes 202 that are connected to a patient. An ECG front end circuit 203 is in electrical communication with the input 2141 via a connector plug and socket or the like. The ECG front end circuit 203 operates to amplify, buffer, filter and optionally digitize an electrical ECG signal generated by the patient's heart to produce a stream of digitized ECG samples. The digitized ECG samples are provided to a controller 204, which can be a processor that combines a DSP and ARM processor. One exemplary controller is the family of Applications Processors manufactured by Texas Instruments Incorporated Inc. In one embodiment of the apparatus, the DSP conducts all of the previously described filtering under the ART protocol, and then passes the multiple streams of filtered ECG data to the ARM processor. The ARM buffers the stream of digitized ECG signal data into segments (buffers) corresponding to a predetermined time. The ARM performs an outcomes analysis on the filtered ECG data to detect VF, shockable VT or other shockable cardiac rhythms. In accordance with the present disclosure, the ARM uses the outcomes analysis to determine a treatment regimen which is most beneficial to the patient. These controller 204 portions of the DSP and ARM thus operate together as an ECG analyzer. Of course, the scope of the present disclosure is not limited to a particular DSP/ARM configuration. The foregoing and following functions can be equivalently implemented in a single processor or distributed among multiple processors.

The ECG analyzer incorporates an analysis algorithm that can determine a shockable cardiac rhythm in the presence of CPR-related signal noise artifact with a defined sensitivity and specificity. The accuracy of the ECG analyzer is sufficient to safely and effectively assess the cardiac state of the input signal in the presence of CPR compressions noise. One such analysis algorithm is ART as described previously.

If the ECG analyzer determines a shockable cardiac rhythm in combination with the determination of a treatment regimen that indicates the need for a defibrillation shock, then controller 204, responsive to the output of the ECG analyzer, sends a signal to a HV (high voltage) charging circuit 2145 to charge a HV energy storage source 206 in preparation for delivering a shock. When the HV energy storage source 206 is fully charged, controller 204 directs a shock button 210 to begin flashing to re-direct the attention of the user from the task of providing CPR compressions to the task of delivering electrotherapy.

As will be described in more detail, controller 204 can initiate the preparation for a defibrillating shock immediately upon detection of a shockable cardiac rhythm, i.e. in a continuous mode of operation, and issue instructions to interrupt of CPR compressions for electrotherapy as soon as the device is armed. Alternatively, the controller 204 can initiate preparation for a defibrillating shock preceding the end of a predetermined period of CPR compressions, and can instruct the immediate delivery of electrotherapy simultaneously with the end of the predetermined period. This last mode is called a scheduled operation mode.

In either continuous or scheduled operation mode, controller 204 controls the user interface 213 to issue aural prompts to terminate CPR and press the shock button to deliver a defibrillating shock. These prompts should be issued together and in quick order so that delay between stopping CPR and pressing the shock button is minimized. The user interface 213 should similarly issue an aural prompt via audio speaker 214 to resume CPR as soon as possible after the controller 204 senses that a defibrillating shock has been delivered, e.g. by sensing the button press, current flow from the HV storage circuit, etc. corresponding visual prompts can be issued simultaneously with the aural prompts.

When the user presses the shock button 210, a defibrillation shock is delivered from HV energy storage source 206 through a Shock delivery circuit 207. In a preferred embodiment, Shock delivery circuit 207 is electrically connected via an output of the AED to the same electrodes 202 which receive the raw ECG signal.

Controller 204 also provides control of the user interface (UI) output functions in the device. The user interface 213 is the primary means for guiding the user through the progress of the cardiac rescue protocol, and so includes at least one of an aural instruction output and a visual display. In particular, user interface 213 may comprise an audio speaker 214 to issue an aural verbal or signal prompt to the user regarding a state of the rescue, an instruction as to a next step to be taken in the rescue, or regarding instructions responsive the determined shockable cardiac rhythm. User interface 213 may also convey audible information via a beeper 209. User interface 213 may also provide visual text or graphical indications on a display 215. User interface 213 may also convey visual information via flashing light LED 212, which may illuminate adjacent graphics or buttons to be pressed. Preferably, controller 204 controls the user interface such that each of these cues is provided in a manner that optimizes the desired response of the user.

Audible and visual cues pertaining to the same information need not be issued simultaneously if one or the other cue may detract from the desired response. For example, controller 204 may control the charging circuit to fully charge the HV storage source to the armed state preceding issuing any instructions at all. Alternatively, controller 204 may drive the user interface to indicate a determination of a shockable cardiac rhythm on visual display 215 preceding issuing related aural instructions on speaker 214. And again, controller 204 may drive the user interface to indicate the state of the HV charging circuit preceding issuing related aural instructions on speaker 214.

Software instructions for operating controller 204 are disposed in an onboard memory. Instructions in non-volatile memory may include the algorithm for the ART algorithm, the algorithm for PAS, instructions for a CPR rescue protocol that includes a period for providing CPR compressions, UI configurations for multiple user types, and the like. Volatile memory may include software-embodied records of device self-tests, device operating data, and rescue event audio and visual recordings.

Other optional features of the defibrillator shown in FIG. 5 include a system monitor controller which receives signals from various Buttons (e.g. Power On, Shock) and provides signals for the beeper and LED lights. State changes of the buttons and sensors are transmitted back to the controller 204 through a communications interface. This feature enables very low-power standby operations with wake-up sensing by means of the button actuation and readiness status outputs.

Furthermore, additional optional features include communication links with a CPR monitor 340 or a CPR mechanical device 350 as known in the art of the present disclosure.

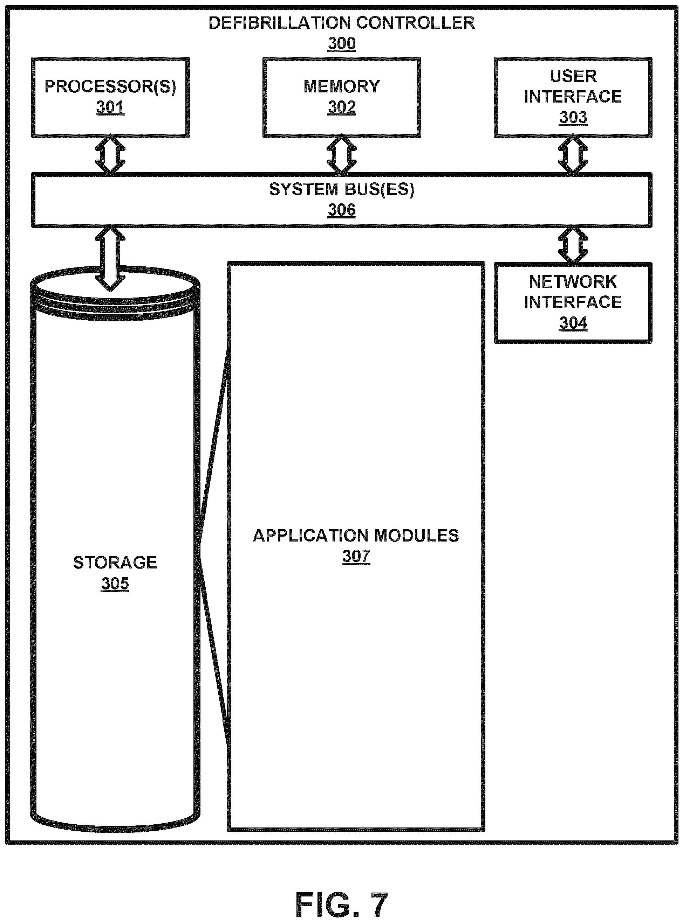

Referring to FIG. 7, shown is an exemplary embodiment of controller 300 that includes one or more processor(s) 301, memory 302, a user interface 303, a network interface 304, and a storage 305 interconnected via one or more system bus(es) 306.

Each processor 301 can be any hardware device, as known in the art of the present disclosure or hereinafter conceived, capable of executing instructions stored in memory 302 or storage or otherwise processing data. In a non-limiting example, the processor(s) 301 can include a microprocessor, field programmable gate array (FPGA), application-specific integrated circuit (ASIC), or other similar devices.

The memory 302 can include various memories, as known in the art of the present disclosure or hereinafter conceived, including, but not limited to, L1, L2, or L3 cache or system memory. In a non-limiting example, the memory 302 can include static random access memory (SRAM), dynamic RAM (DRAM), flash memory, read only memory (ROM), or other similar memory devices.

In practice, controller 300 also provides control of the user interface (UI) output functions. Specifically, user interface 303 is the primary means for guiding the responder through the protocols of the present disclosure, and so includes at least one of an aural instruction output and a visual display. In particular, user interface 303 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). User interface 303 can also convey audible information via a beeper. User interface 303 can also provide visual text or graphical indications on a display. User interface 303 can also convey visual information via a flashing light LED, which may illuminate adjacent graphics or buttons to be pressed. Preferably, controller 300 controls the user interface 301 such that each of these cues is provided in a manner that optimizes the desired response of the responder in the execution of protocols of the present disclosure.

Still referring to FIG. 7, network interface 304 can include one or more devices, as known in the art of the present disclosure or hereinafter conceived, for enabling communication with other components of defibrillator (defibrillator 200 of FIG. 6) or another device, particularly a mechanical CPR device or a CPR coaching device, as known in the art of the present disclosure or hereinafter conceived, in the administration of CPR/chest compression to a patient in accordance with the protocols of the present disclosure and/or in the acquisition of CPR data indicative of the quality of CPR being administered to the patient.

In a non-limiting example, the network interface 304 can include a network interface card (NIC) configured to communicate according to the Ethernet protocol. Additionally, the network interface 414 may implement a TCP/IP stack for communication according to the TCP/IP protocols. Various alternative or additional hardware or configurations for the network interface 304 will be apparent.

The storage 305 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 305 can store instructions for execution by the processor(s) 301 or data upon with the processor(s) 301 may operate. For example, the storage 305 may store a base operating system for controlling various basic operations of the hardware.

The storage 305 can also store an application modules 307 in the form of executable software/firmware for implementing the methods of the present disclosure as previously described in the present disclosure.

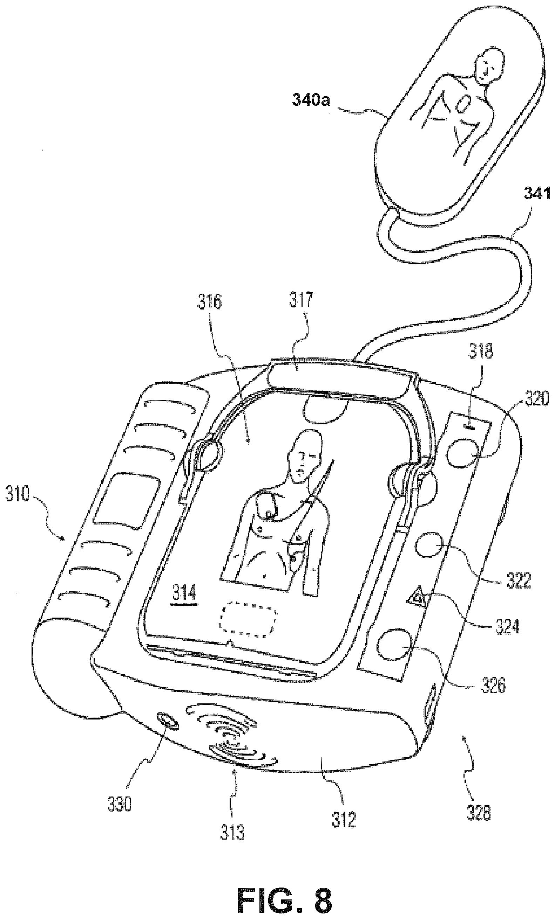

FIG. 8 illustrates the CPR coaching device 100 coupled through cable 130 to the defibrillator 310. The defibrillator 310 represents a semi-automatic external defibrillator (AED). However, other types of defibrillators can be used as well. The AED 310 is housed in a rugged polymeric case 312 which protects the electronic circuitry inside the case, which was previously described with reference to FIG. 5, and also protects the responder 220 from shocks. Attached to the case 312 by electrical leads are a pair of electrodes 316. The electrodes 316 are housed in a cartridge 314 located in a recess on the top side of the AED 310. The electrode pads are accessed for use by pulling up on a handle 317 which allows removal of a plastic cover over the electrodes 316. The user interface is on the right side of the AED 310. A small ready light 318 informs the responder 220 of the readiness of the AED 310. In this embodiment the ready light blinks after the AED 310 has been properly set up and is ready for use. The ready light is on constantly when the AED 310 is in use, and the ready light is off or flashes in an alerting color when the AED 310 needs attention.

Below the ready light is an on/off button 320. The on/off button is pressed to turn on the AED 310 for use. To turn off the AED 310 the responder 220 holds the on/off button down for one second or more. An information button 322 flashes when information is available for the responder 220. The responder 220 depresses the information button to access the available information, which is then presented as an audible message. A caution light 324 blinks when the AED 310 is acquiring heartbeat information from the patient 210 and lights continuously when a shock is advised, alerting the responder 220 and others that no one should be touching the patient 210 during these times. A shock button 326 is depressed to deliver a shock after the AED 310 informs the responder 220 that a shock is advised. An infrared port 328 on the side of the AED 310 is used to transfer data between the AED 310 and a computer. This data port finds used after the patient 210 has been rescued and a physician desires to have the AED 310 event data downloaded to his or her computer for detailed analysis. A speaker 313 provides voice instructions to the responder 220 to guide the responder 220 through the use of the AED 310 to treat the patient 210. A beeper 330 is provided which "chirps" when the AED 310 needs attention such as electrode pad replacement or a new battery. The beeper can also be used as a metronome tone which chirps at the appropriate rate of CPR chest compressions.

In another embodiment the CPR coaching device includes ECG electrodes on the body-contacting surface of the device for the sensing of the patient's ECG signal. The ECG signal detected by the CPR coaching device is coupled to the ECG front end circuit 202 for processing. In one implementation the CPR coaching device of this embodiment is applied to the patient's chest before the usual defibrillator electrodes are unwrapped and applied. The ECG sensor on the coaching device can thereby give the defibrillator a "quick look" at the patient's ECG waveform. For instance, if the ECG signals is sensed and processed to determine that the patient exhibits a viable ECG signal, the defibrillator can alert the responder that defibrillation is not advised for the patient. The responder does not need to unwrap and apply the defibrillation electrodes to the patient and another form of therapy can be recommended by the defibrillator such as CPR.

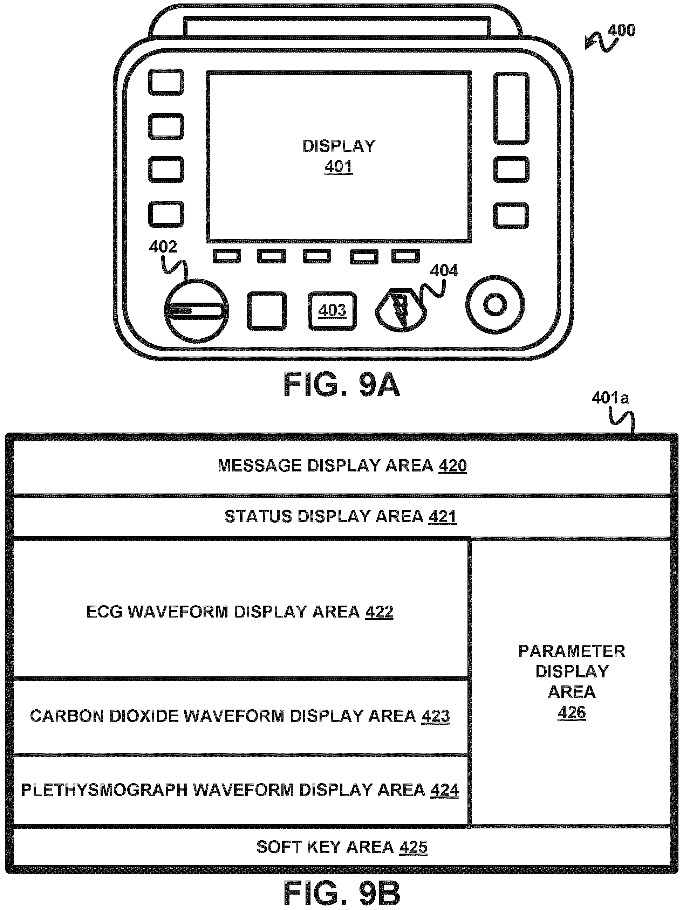

Referring to FIGS. 9A and 9B, an exemplary monitor/defibrillator 400 of the present disclosure that is utilized to shock a patient experiencing cardiac arrest in an attempt to restore a heart of the patient to a normal cardiac rhythm as would be appreciated by those having ordinary skill in the art. As shown, monitor/defibrillator 400 incorporates a settings dial 402 for enabling a clinician to set various parameters of the shock (e.g., an energy level of the shock), a charge control 403, a shock control 404 and a display 401.

In accordance with the present disclosure, an exemplary monitor display screen 404a includes a message display area 420, a status display area 421, an ECG waveform display area 422, a carbon dioxide waveform display area 423, a plethysmograph waveform display area 424, a soft key area 425 and a parameter display area 425 as known in the art of the present disclosure.

In practice, various technical features for an ALS monitor/defibrillator 400 can be used in manual mode of operation in a resuscitation setting. Specifically, the features pertain to the display of resuscitation information on monitor/defibrillator 400 device to the ALS responder to help make clinical decisions during resuscitation efforts and improve the quality of care.

In one exemplary embodiment, monitor/defibrillator 400 incorporates ART, an ECG analysis algorithm that can detect a cardiac arrythmia in the presence of artifact induced by chest compressions, in a manual mode of operation, and monitor/defibrillator 400 further incorporates a display of the shock outcome (successful or unsuccessful) in display area 426 within 10-15 seconds post-shock using the ART algorithm's output.

In a second exemplary embodiment, monitor/defibrillator 400 incorporates a display of the cumulative time in VF after defibrillation has been identified by ART in display area 426.

In a third exemplary embodiment, monitor/defibrillator 400 incorporates a CPR feedback device/algorithm, and further incorporates a display of continuously updated chest compression fraction (CCF) in display area 426 using the output from a CPR feedback device/algorithm.

In a fourth exemplary embodiment, monitor/defibrillator 400 incorporates a display of the presenting rhythm in the resuscitation (shockable vs. non-shockable) and further incorporates a communication capability with an AED to extract the presenting rhythm from the AED.

In a fourth exemplary embodiment, monitor/defibrillator 400 incorporates a display of the total number of shocks delivered in conjunction with the rhythm shockability at different part resuscitation (e.g., Shockable cardiac rhythm... shockable cardiac rhythm....shock delivered (1^{st} shock) .....non-shockable cardiac rhythm..... shockable cardiac rhythm....shock applied (1^{st} shock after NS rhythm, 2 total shocks).

The technical features described above provide critical information to ALS rescuers to help make clinical decisions during resuscitation efforts and improve the quality of care.

Display of the shock outcome is meaningful to the responder as it can help distinguish between refractory and recurrent VF, which can be used to help guide clinical decision making, such as epinephrine administration or double-sequential defibrillation.

Display of the cumulative time in VF helps the responder assess whether they should interrupt CPR to shock right away or continue with CPR to ensure adequate amounts of CPR have been provided to improve VF vitality.

Display of the presenting rhythm shockability provides the responder with information about whether to continue resuscitation efforts. Presenting non-shockable cardiac rhythms may support termination of resuscitation decisions, whereas presenting shockable cardiac rhythms may support continuing the resuscitation. If an AED was used preceding the ALS device, this feature would work best if the ALS device can capture the presenting rhythm information from the AED.

Display of the total number of shocks delivered in conjunction with the rhythm shockability at different parts of the resuscitation provides the responder with a timeline of events within the resuscitation. An example of the display could be: Shockable cardiac rhythm... shockable cardiac rhythm....shock delivered (1^{st} shock) .....non-shockable cardiac rhythm.....shockable cardiac rhythm....shock applied (1^{st} shock after NS rhythm, 2 total shocks).

This information helps guide clinical decision making in multiple ways. It not only provides insight to the total number of shocks provided, but also provides information about shock efficacy, which helps the responder distinguish between refractory vs. recurrent VF.

This information can also be made available in retrospective review software (like EventReview Pro).

From the description of FIGS. 1-9B herein, those having ordinary skill in the art will appreciate the numerous benefits of the present disclosure including, but not limited to, a mode management of systematically operating a defibrillator between a scheduled operation mode and a custom operation mode by disabling execution(s) of the custom operation mode of the defibrillator preceding delivery of an initial defibrillating shock to the heart of the patient and enabling execution(s) of the custom operation mode of the defibrillator succeeding the delivery of the initial defibrillating shock to the heart of the patient.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which can be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as can be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A defibrillation controller (204), comprising:
a non-transitory machine-readable storage medium (302) encoded with instructions for execution by at least one processor (301), the non-transitory machine-readable storage medium (302) including the instructions to:
systematically operate a defibrillator (200) between a scheduled operation mode and a custom operation mode,
wherein the scheduled operation mode includes:
an uninterruptible CPR protocol specifying at least one rule/guideline for a guidance by the defibrillator (200) of an administration of a cardiopulmonary resuscitation to a heart of patient over a fixed CPR time period of the uninterruptible CPR protocol; and
a scheduled shock protocol specifying at least one rule/guideline for a conditional delivery of a defibrillating shock to the heart of the patient by the defibrillator (200) subsequent to an expiration of the fixed CPR time period, and
wherein the custom operation mode includes:
an interruptible CPR protocol specifying at least one rule/guideline for a guidance by the defibrillator (200) of the administration of the cardiopulmonary resuscitation to the heart of patient over a variable time period; and
a custom shock protocol specifying at least one rule/guideline for the conditional delivery of the defibrillating shock to the heart of the patient by the defibrillator (200) subsequent to an expiration of the variable time period of the interruptible CPR protocol;
wherein an initial execution of the custom operation mode by the defibrillator (200) is disabled prior to a delivery of an initial defibrillating shock to the heart of the patient by the defibrillator (200); and
wherein the initial execution of the custom operation mode by the defibrillator (200) is enabled subsequent to the delivery of the initial defibrillating shock to the heart of the patient by the defibrillator (200).

2. The defibrillation controller (204) of claim 1, wherein the non-transitory machine-readable storage medium (302) further includes instructions to:
execute a shock-first operation mode by the defibrillator (200) prior to systematically operating the defibrillator (200) between the scheduled operation mode and the custom operation mode, wherein the shock-first operation mode includes one of a F-Shock Advisory; and
enable the initial execution of the custom operation mode by the defibrillator (200) responsive to the delivery of the initial defibrillating shock to the heart of the patient by the defibrillator (200) during the execution of the shock-first operation mode by the defibrillator (200).

3. The defibrillation controller (204) of claim 1, wherein the non-transitory machine-readable storage medium (302) further includes the instructions to:
execute a CPR-first operation mode by the defibrillator (200) prior to systematically operating the defibrillator (200) between the scheduled operation mode and the custom operation mode, wherein the CPR-first operation mode includes one of a C-Shock Advisory; and
enable the initial execution of the custom operation mode by the defibrillator (200 responsive to the delivery of the initial defibrillating shock to the heart of the patient by the defibrillator (200) during the execution of the CPR-first operation mode by the defibrillator (200).

4. The defibrillation controller (204) of claim 1, wherein, when the initial execution of the custom operation mode by the defibrillator (200) is enabled, the non-transitory machine-readable storage medium (302) further includes the instructions to:
initiate an execution of the interruptible CPR protocol by the defibrillator (200) including a C-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the defibrillator (200) when the C-Shock Advisory derives the shock decision from the electrocardiogram prior to an expiration of a maximum time period for the execution of the interruptible CPR protocol.

5. The defibrillation controller (204) of claim 1, wherein, when the initial execution of the custom operation mode by the defibrillator (200) is enabled, the non-transitory machine-readable storage medium (302) further includes the instructions to:
initiate an execution of the interruptible CPR protocol by the defibrillator (200) including a C-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the defibrillator (200) when the C-Shock Advisory derives the shock decision from the electrocardiogram subsequent to an expiration of a minimum time period for the execution of the interruptible CPR protocol and prior to an expiration of a maximum time period for the execution of the interruptible CPR protocol.

6. The defibrillation controller (204) of claim 1, wherein, when the initial execution of the custom operation mode by the defibrillator (200) is enabled, the non-transitory machine-readable storage medium (302) further includes the instructions to:
initiate an execution of the interruptible CPR protocol by the defibrillator (200) including:
a C-Shock Advisory of an electrocardiogram of the heart of the patient to derive a shock decision or a no-shock/undecided decision; and
a CPR Quality Analysis of the execution of the interruptible CPR protocol to derive a compliant CPR decision or a non-compliant CPR decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the defibrillator (200) when the C-Shock Advisory or the CV-Shock Advisory derives the shock decision from the electrocardiogram subsequent prior to an expiration of a maximum time period for the execution of the interruptible CPR protocol and when the CPR Quality Analysis derives the non-compliant CPR decision prior to the expiration of the maximum time period for the execution of the interruptible CPR protocol.

7. The defibrillation controller (204) of claim 1, wherein, when the initial execution of the custom operation mode by the defibrillator (200) is enabled, the non-transitory machine-readable storage medium (302) further includes the instructions to:
initiate an execution of the interruptible CPR protocol by the defibrillator (200) including a C-Shock Advisory or a CV-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the defibrillator (200) when the C-Shock Advisory or the CV-Shock Advisory derives the shock decision from the electrocardiogram prior to an expiration of a maximum time period for the execution of the interruptible CPR protocol and prior to exceeding a maximum number of a plurality of defibrillating shocks to the heart of the patient.

8. A defibrillator (200), comprising:
a shock delivery circuit (207) operable to deliver at least one defibrillating shock to a heart of a patient; and
a defibrillation controller (204) configured to systematically operate the defibrillator (200) between a scheduled operation mode and a custom operation mode,
wherein the scheduled operation mode includes:
an uninterruptible CPR protocol specifying at least one rule/guideline for a guidance by the defibrillator (200) of an administration of a cardiopulmonary resuscitation to a heart of patient over a fixed CPR time period of the uninterruptible CPR protocol; and
a scheduled shock protocol specifying at least one rule/guideline for a conditional delivery of the defibrillating shock to the heart of the patient by the shock delivery circuit (207) subsequent to an expiration of the fixed CPR time period, and
wherein the custom operation mode includes:
an interruptible CPR protocol specifying at least one rule/guideline for a guidance by the defibrillator (200) of the administration of the cardiopulmonary resuscitation to the heart of patient over a variable time period; and
a custom shock protocol specifying at least one rule/guideline for the conditional delivery of the defibrillating shock to the heart of the patient by the shock delivery circuit (207) subsequent to an expiration of the variable time period of the interruptible CPR protocol;
wherein an initial execution of the custom operation mode by the defibrillator (200) is disabled prior to a delivery of an initial defibrillating shock to the heart of the patient by the shock delivery circuit (207); and
wherein the initial execution of the custom operation mode by the defibrillator (200) is enabled subsequent to the delivery of the initial defibrillating shock to the heart of the patient by the shock delivery circuit (207).

9. The defibrillator (200) of claim 8, wherein the defibrillation controller (204) is further configured to:
execute a shock-first operation mode by the defibrillator (200) prior to systematically operating the defibrillator (200) between the scheduled operation mode and the custom operation mode, wherein the shock-first operation mode includes one of a F-Shock Advisory; and
enable the initial execution of the custom operation mode responsive to the delivery of the initial defibrillating shock to the heart of the patient by the shock delivery circuit (207) during the execution of the shock-first operation mode by the defibrillator (200).

10. The defibrillator (200) of claim 8, wherein the defibrillation controller (204) is further configured to:
execute a CPR-first operation mode prior to systematically operating the defibrillator (200) between the scheduled operation mode and the custom operation mode, wherein the CPR-first operation mode includes one of a C-Shock Advisory; and
enable the initial execution of the custom operation mode responsive to the delivery of the initial defibrillating shock to the heart of the patient during the execution of the CPR-first operation mode by the defibrillator (200).

11. The defibrillator (200) of claim 8, wherein, when the initial execution of the custom operation mode by the defibrillator (200) is enabled, the defibrillation controller (204) is further configured to:
initiate an execution of the interruptible CPR protocol by the defibrillator (200) including a C-Shock Advisory or a CV-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the shock delivery circuit (207) when the C-Shock Advisory or the CV-Shock Advisory derives the shock decision from the electrocardiogram prior to an expiration of a maximum time period for the execution of the interruptible CPR protocol.

12. The defibrillator (200) of claim 8, wherein, when the initial execution of the custom operation mode by the defibrillator (200) is enabled, the defibrillation controller (204) is further configured to:
initiate an execution of the interruptible CPR protocol by the defibrillator (200) including a C-Shock Advisory or a CV-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the shock delivery circuit (207) when the C-Shock Advisory or the CV-Shock Advisory derives the shock decision from the electrocardiogram subsequent to an expiration of a minimum time period for the execution of the interruptible CPR protocol and prior to an expiration of a maximum time period for the execution of the interruptible CPR protocol.

13. The defibrillator (200) of claim 8, wherein, when the initial execution of the custom operation mode by the defibrillator (200) is enabled, the defibrillation controller (204) is further configured to:
initiate an execution of the interruptible CPR protocol by the defibrillator (200) including:
a C-Shock Advisory or a CV-Shock Advisory of an electrocardiogram of the heart of the patient to derive a shock decision or a no-shock/undecided decision; and
a CPR Quality Analysis of the administration of the CPR to the heart of the patient to derive a compliant CPR decision or a non-compliant CPR decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the shock delivery circuit (207) when the C-Shock Advisory or the CV-Shock Advisory derives the shock decision from the electrocardiogram subsequent prior to an expiration of a maximum time period for the execution of the interruptible CPR protocol and when the CPR Quality Analysis derives the non-compliant CPR decision prior to the expiration of the maximum time period for the execution of the interruptible CPR protocol.

14. The defibrillator (200) of claim 8, wherein, when the initial execution of the custom operation mode by the defibrillator (200) is enabled, the defibrillation controller (204) is further configured to: initiate an execution of the interruptible CPR protocol by the defibrillator (200) including a C-Shock Advisory or a CV-Shock Advisory of an electrocardiogram of the heart of the patient to derive a shock decision or a no-shock/undecided decision; and
interrupt the execution of the interruptible CPR protocol by the defibrillator (200) and initiate an execution of the custom shock protocol by the defibrillator (200) including a C-Shock Delivery of a delivery of an additional defibrillation shock to the heart of the patient by the shock delivery circuit (207) when the C-Shock Advisory or the CV-Shock Advisory derives the shock decision from the electrocardiogram prior to an expiration of a maximum time period for the execution of the interruptible CPR protocol and prior to exceeding a maximum number of a plurality of defibrillating shocks to the heart of the patient.

15. A defibrillation method, comprising:
systematically operating a defibrillator (200) between a scheduled operation mode and a custom operation mode,
wherein the scheduled operation mode includes:
an uninterruptible CPR protocol specifying at least one rule/guideline for a guidance by the defibrillator (200) of an administration of a cardiopulmonary resuscitation to a heart of patient over a fixed CPR time period of the uninterruptible CPR protocol; and
a scheduled shock protocol specifying at least one rule/guideline for a conditional delivery of a defibrillating shock to the heart of the patient by the defibrillator (200) subsequent to an expiration of the fixed CPR time period, and
wherein the custom operation mode includes:
an interruptible CPR protocol specifying at least one rule/guideline for a guidance by the defibrillator (200) of the administration of the cardiopulmonary resuscitation to the heart of patient over a variable time period; and
a custom shock protocol specifying at least one rule/guideline for the conditional delivery of the defibrillating shock to the heart of the patient by the defibrillator (200) subsequent to an expiration of the variable time period of the interruptible CPR protocol;
wherein an initial execution of the custom operation mode by the defibrillator (200) is disabled prior to a delivery of an initial defibrillating shock to the heart of the patient by the defibrillator (200); and
wherein the initial execution of the custom operation mode by the defibrillator (200) is enabled subsequent to the delivery of the initial defibrillating shock to the heart of the patient by the defibrillator (200).
